# EUROPEAN PATENT APPLICATION

(11) **EP 3 699 578 A1**
(43) Date of publication of application: **26.08.2020**
(21) Application number: 19158307.9
(22) Date of filing: 20.02.2019
(51) Int. Cl.: G01N 21/88, D03J 1/00, D06H 3/08, G01N 21/898, G01N 33/36, G01N 33/44, D06H 3/02, G01N 21/89

(54) **SYSTEM FOR IMPROVED FABRIC INSPECTION**

(71) Applicant: Comelz S.p.A., 27029 Vigevano (IT)
(72) Inventor: CORSICO PICCOLINO, Alessandro, 27029 Vigevano (PV) (IT)
(74) Representative: Botti, Mario

(57) **Abstract**

A system (1) for fabric inspection, for example for footwear, leather goods, clothing, automotive components and the like, comprises a main body (1') provided with connection means (1c) for the connection thereof to a pre-existing inspection frame on which fabrics to be inspected are arranged, a plurality of image detectors (2) included in the main body (1') and adapted to acquire images of a fabric that is moved by the inspection frame, a processing unit (3) of the images captured by the image detectors (2), this processing unit (3) being adapted to generate a digital copy of the inspected fabric, and a signal element (5) adapted to indicate defects on the fabric surface, wherein the processing unit (3) is configured to correlate the defects on the fabric indicated by the signal element (5) with the captured image acquired of the fabric, generating a report indicating at least the shape and the position of the defects on the fabric.

## Description

### Technical field

The present invention refers to a system, and to a corresponding method, for fabric inspection, for example fabrics for leather goods, footwear, clothing, automotive industry and the like, and the following description is made with reference to this application field with the only purpose of simplifying the exposition thereof.

### Prior art

As it is well known in this specific technical field, every type of fabric, even those of the best quality, can present surface defects, such as small stains, loose threads, missing threads and the like.

For this reason, in fabric quality control, it is important to identify and report all the possible defects before the subsequent processing of such a fabric.

To that aim, there are specific inspection frames on which the fabrics to be inspected are arranged, such fabrics being wrapped in rolls. Specifically, during the inspection, the fabrics are unwrapped from the frame so as to allow an operator to detect all the defects. Once the defect is identified, the operator stops the movement of the fabric and places a specific signal element such as a label, at the defect. The operator has also the task of drafting a report of the performed inspection, which report is to be shown, for example, to the fabric producer or to the company in charge of the further processing of such a fabric.

Therefore, all the operations that lead to the identification of the defect and to the drafting of the final report are performed exclusively by a specialized operator, with the consequent reduction of the inspection efficiency. Furthermore, the known solution described above does not allow to know the exact surface of the fabric that is wasted due to the defects, as well as it does not allow to obtain a detailed report of such defects. Further, in this known solution, the drafting of the final report may be compromised by errors of the operator, while possible objections in relation to the defects, for example objections addressed to the producer of the defected fabric, are very complex to handle. Last, the manual drafting of the report increases fabric inspection times.

Consequently, there is the need of a system that allows to identify and report the defects of a fabric in a more efficient and quick manner.

The technical problem of the present invention is to provide a system for fabric inspection having features so as to allow overcoming the limitations and drawbacks which still affect the known solutions, in particular able to improve the inspection of fabrics that is carried out by operators on the inspection frames, increasing the efficiency and the reliability of such inspection.

### Summary of invention

The solution idea at the basis of the present invention is to provide a system capable of being associated with pre-existing inspection frames and capable of capturing a complete digital image of the fabric that is moved in such frames. The system further provides means that allow an operator to indicate the defects present on the fabric without marking the surface thereof. In this way, the system of the present invention is capable of keeping in its memory the captured image of the fabric, together with a detailed report of the defects of such fabric as indicated by the operator, wherein for each defect is automatically reported the type, the entity and the exact position on the fabric according to the indications provided by the operator.

On the basis of such idea of solution, the above technical problem is solved by a system for fabric inspection comprising a main body provided with connection means for the connection thereof to a pre-existing inspection frame on which fabrics to be inspected are arranged, a plurality of image detectors included in the main body and adapted to capture images of a fabric that is moved by the inspection frame, a processing unit of the images captured by the image detectors, this processing unit being adapted to generate a digital copy of the inspected fabric, and a signal element adapted to indicate defects on the fabric surface, wherein the processing unit is configured to correlate the defects on the fabric indicated by the signal element with the captured image of the fabric, generating a report indicating at least the shape and the position of the defects on the fabric.

More particularly, the invention comprises the following additional characteristics, taken individually or in combination if required.

According to an aspect of the present invention, the signal element can be provided with a lighting element capable of being detected by the image detectors, the coordinates of the movement of the lighting element being calculated by means of the processing unit.

In particular, the processing unit can be configured to recognize the position, the shape and/or the entity of the defect of the fabric surface on the basis of said coordinates of the movement of the lighting element.

According to an aspect of the present invention, the signal element can be configured to automatically recognize specific gestures performed by the operator, in such a way to drive the system by means of said specific gestures.

According to an aspect of the present invention, the main body can comprise a plurality of lighting elements adapted to illuminate the surface of the fabric to be inspected.

According to another aspect of the present invention, the processing unit can be a unit external to the main body and operatively connected therewith.

According to another aspect of the present invention, the processing unit can be operatively connected with a memory unit adapted to store at least the digitalized image of the inspected fabric and the generated report indicating the defects of the fabric.

According to another aspect of the present invention, the memory unit can be a cloud unit which is accessed from remote by a user through a specific application and/or a specific address.

Furthermore, the processing unit can be configured to define on the digitalized image a plurality of cutting areas, the type, the number and the position of such cutting areas being defined and optimized on the basis of the information acquired by the processing unit during fabric inspection.

According to another aspect of the present invention, the processing unit can be configured to recognize patterns or geometric shapes that are repeated in the fabric, the report further showing the variance in the pattern of the real fabric with respect to that of the theoretical fabric.

According to another aspect of the present invention, the connection means can be configured to connect the main body of the system to the pre-existing frame in a removable manner.

According to yet another aspect of the present invention, the fabric to be inspected can be a fabric of the type for footwear, leather goods, clothing components and the like, wrapped in rolls on the inspection frame, which is configured to unwrap/wrap said fabric during the inspection.

According to yet another aspect of the present invention, the main body can comprise a user interface.

Furthermore, the main body can comprise a laser projector adapted to reproduce on the fabric the outline of the defect reported through the signal element, providing the operator with a feedback.

Finally, the signal element can be in the form of labels applied at the defect of the fabric to be inspected, said labels being identified by the image detectors and containing information about the type/entity of the defect.

The present invention also refers to an apparatus for fabric inspection comprising means (for example, a frame) adapted to hold and move a fabric to be inspected, said apparatus including an inspection system as indicated above.

The present invention also refers to a method for fabric inspection, for example for footwear, leather goods, clothing components and the like, comprising the steps of connecting a plurality of image detectors to a pre-existing inspection frame, on which fabrics to be inspected are arranged, capturing, by means of the image detectors, images of the fabric moved by the inspection frame, generating a digital copy of the inspected fabric by means a processing unit of the images captured by the image detectors, signaling defects on the fabric surface through a signal element, correlating, through the processing unit, the defects on the fabric indicated by the signal element with the captured image of the fabric, and generating a report indicating the shape and the position of the defects on the fabric.

In particular, the signaling of the defects can occur by means of a lighting element or by means of labels.

The features and advantages of the system and method according to the invention will become apparent from the following description of an embodiment thereof, given by way of non-limiting example with reference to the accompanying drawings.

### Brief description of the drawings

In such drawings:
- figure 1 schematically shows the inspection system according to the present invention;
- figure 2 is a general schematic representation of the system of the present invention applied to an inspection frame;
- figures 3A and 3B show, respectively, a lateral view and a top view of an inspection frame on which the system of the present invention is assembled; and
- figure 4 is an example of a report generated by the system of the present invention, highlighting the type and the position of the defects identified on the fabric.

### Detailed description

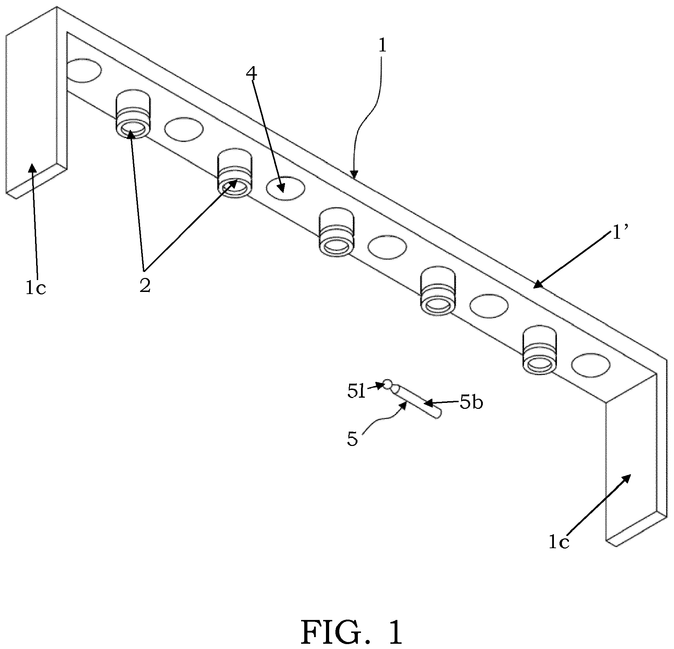

With reference to those figures, and in particular to figure 1, a system for fabric inspection according to the present invention is globally and schematically indicated with the reference number 1, such system being adapted to execute a corresponding method.

It is worth noting that the figures represent schematic views and are not drawn to scale, but instead they are drawn so as to emphasize the important features of the invention. Moreover, in the figures, the different elements are depicted in a schematic manner, their shape varying depending on the application desired. It is also noted that in the figures the same reference numbers refer to elements that are identical in shape or function. Finally, particular features described in relation to an embodiment illustrated in a figure are also applicable to the other embodiments illustrated in the other figures.

In its most general form, the system 1 allows to carry out, in a particularly efficient manner, the inspection of fabrics arranged in rolls on an inspection frame. As an example, the fabrics to be inspected can be fabrics for footwear, leather goods, clothing, automotive components and the like, even if the present invention is not limited to a certain type of fabric. Preferably, the present invention is addressed to fabrics of the textile industry, but the application thereof also to sheets of hide/leather material is not excluded. Consequently, the preferred application is in relation to fabrics for the textile industry that can possibly be cut subsequently for the realization of single items, even if, as illustrated above, other applications are not excluded.

As illustrated in the schematic view of figure 1, the system 1 of the present invention comprises, first of all, a main body 1' that houses its main components.

In particular, in an embodiment of the present invention, the main body 1' has an elongated shape with square or rectangular section, even if other shapes are obviously possible and the present invention is not limited to a particular shape for the main body 1'.

The main body 1' houses a plurality of image detectors 2, which allow the capture of images of the surface of a fabric while it is moved, in particular unwrapped/wrapped, by an inspection frame, which is indicated herein with the reference F. In other words, according to the present invention, the fabric to be inspected is a fabric in rolls wrapped on the inspection frame F, which is configured to unwrap/wrap said fabric during the inspection, such fabric being indicated with the reference T in the drawings.

Specifically, the image detectors 2 are photo cameras or video cameras configured to capture high-resolution images of the surface of the fabric to be inspected, such detectors being arranged on the side of the main body 1' facing the fabric.

The image detectors 2 are arranged on a line, or as a grid or matrix, in order to cover a surface that spaces and corresponds at least to the transverse dimension of the fabric to be inspected, namely a dimension that is transversal to the unwrapping direction of the fabric, which is indicated in the figures as H-H direction.

In particular, as it will be illustrated in greater detail below, the image detectors 2 allow to acquire in a continuous manner images of the surface of the fabric during unwrapping thereof, in such a way that an overlapping of the captured images generates a complete digitalized image of the fabric to be inspected.

The image detectors 2 are digital video cameras or photo cameras, for example CCD, and incorporate a processor that transforms and makes immediately available an image data file in a format that is processable by a processing unit, for example a JPEG format or the like.

In a preferred embodiment, three to five cameras are arranged on a face of the main body 1' facing the fabric to be inspected.

Obviously, the skilled in the art well understands that a different numbering or arrangement of the image detectors 2 can be provided; it is only important that, for every instant, the image detectors 2 are capable of acquiring an image of the fabric to be inspected covering at least the whole transverse dimension thereof.

Each image detector 2 is therefore adapted to capture a series of images, one after the other, during the movement of the fabric in the inspection frame, in such a way to generate a digital image of the whole fabric by means of the overlapping of the different captured images.

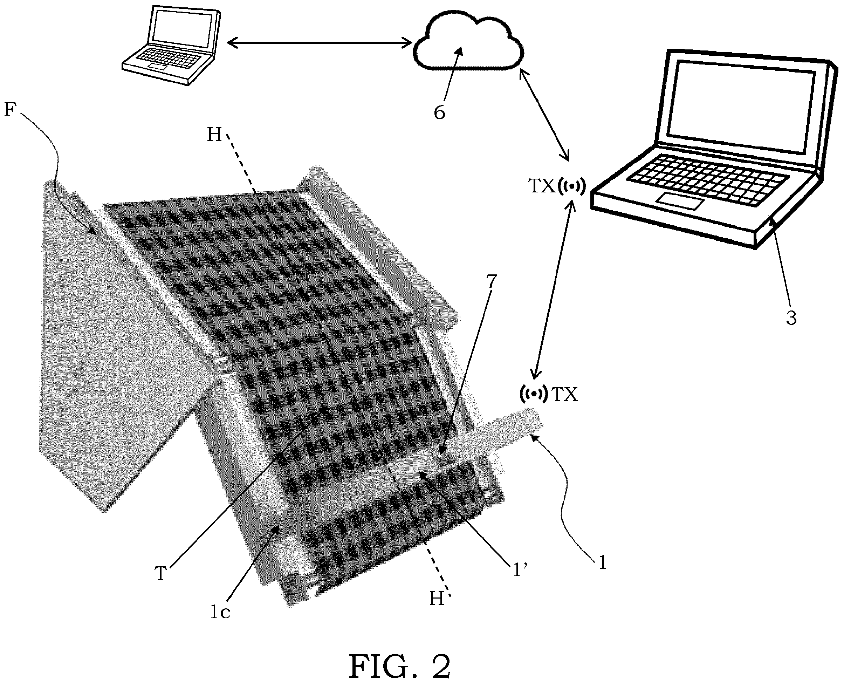

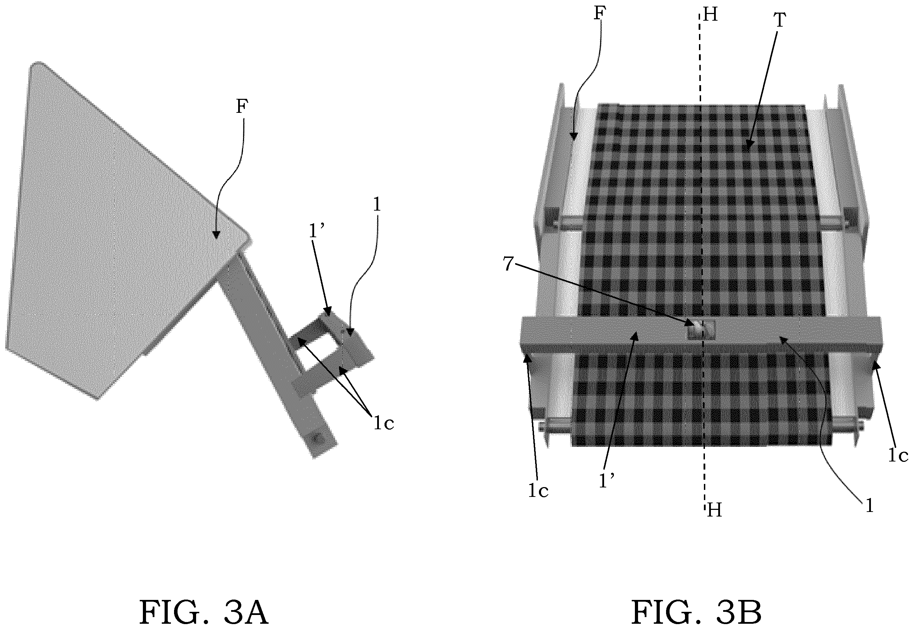

The main body 1', once applied to the inspection frame F, is the crosspiece of such frame, as indicated in the views of figure 2 and of figures 3A and 3B, even if other arrangements can be provided.

Advantageously according to the present invention, the main body 1' is adapted to be associated also with pre-existing inspection frames. In particular, the system 1 of the present invention is provided with connection means 1c that allow it to be associated with pre-existing frames, preferably in a removable manner. For example, the connection means 1c are arms extending from the main body 1' and are provided at the ends thereof with suitable hooks or clamps that allow the connection to the inspection frame F. In this manner, suitably, a pre-existing frame can be easily reconfigured, by means of the simple installation of the main body 1'.

Furthermore, the system 1 comprises a processing unit 3 of the images captured by the image detectors 2.

The processing unit 3 can be integrated inside the main body 1', for example it can be a suitable processor integrated in the main body 1'. Alternatively, in a preferred embodiment of the present invention, the processing unit 3 is a unit external to the main body 1' and operatively connected with it. In this last case, the processing unit 3 can be connected to the main body 1' through various ways, for example by a wire connection or in a wireless way. In any case, both the main body 1' and the processing unit 3 are provided with transmission/reception means TX that allow a data exchange between them.

Suitably, the processing unit 3 is adapted to generate a digital copy of the inspected fabric. Specifically, the processing unit 3 is configured to receive the image data acquired in a continuous manner by the image detectors 2 during the movement of the fabric, such image data being joined and overlapped by the processing unit 3 in such a way as to obtain a complete image of the fabric. In other words, the processing unit 3, starting from the data provided by the image detectors 2, is capable of generating a single file (for example a single .pdf file) containing a high-resolution image of the surface of the fabric to be inspected.

Furthermore, the system 1 comprises a plurality of lighting elements 4 adapted to illuminate the fabric to be inspected. In particular, the lighting elements 4, which are for instance LED lights, are arranged on the same side of the main body 1 on which the image detectors 2 are arranged, in such a way as to illuminate the surface of the fabric to be inspected in a uniform manner; for example, they are arranged between an image detector and the other one.

The system 1 of the present invention also comprises a signal element 5 adapted to signal defects on the fabric surface. In a preferred embodiment, the signal element 5 is shaped so as to be easily grabbed by the operator. The signal element 5 is therefore an instrument that allows the operator to signal the defects identified on the surface of the fabric in a simple and efficient manner.

In particular, in this preferred embodiment, the signal element 5 is provided with an elongated body 5b that represents the grip and with a lighting element 51, such as an ordinary light bulb, connected to an end of the elongated body 5b. The lighting element 51 is such that its light emission is detected by the image detectors 2. In this manner, advantageously, according to the present invention, the coordinates of the movement of the lighting element 51, for example the coordinates of the center of the light bulb, are detected by the system 1, in particular are calculated through the processing unit 3, which processes the information coming from the image detectors 2.

However, the signal element 5 can be also based on a different technology: for example, it could be provided with a RF signaling system, the operation of such signal element 5 not limiting the scope of the present invention.

According to the present invention, once the user identifies a defect on the fabric surface, it stops the movement means of the frame and signals such defect through the signal element 5; in particular, it performs specific movements for each specific defect, for example movements that reproduce the shape of such defect. The processing unit 3 is therefore configured to recognize the position, the shape and/or the entity of the defect on the basis of the coordinates of the movement of the lighting element 51.

By means of the signal element 5 the operator is thus capable of reporting the presence of a defect without marking directly the surface of the fabric, making a non-invasive inspection.

However, according to an embodiment of the present invention, in addition to the signaling through the signal element 5, the operator can arrange on the surface of the fabric specific labels as well, as described in relation with the prior art.

Furthermore, the applied labels can be such as to be readable by the image detectors 2 automatically. Consequently, in an alternative embodiment of the present invention, the operator can signal the defects simply by means of the application of the labels, which can have different shapes and/or colors to indicate different types of defects. In this manner, the system recognizes, by means of the labels, the position and the type of defects. Consequently, in this embodiment, the signal element 5 is represented by the labels and there is no lighting element 51.

Going back now to the preferred embodiment illustrated before, the signal element 5 can be configured to automatically recognize specific gestures performed by the user, in such a way as to drive the system 1 according to such gestures. In this case, the signal element 5 incorporates movement sensors, such as accelerometers, gyroscopes and the like, capable of detecting a movement related to a specific movement made by the user.

In any case, in general, the system 1 is anyway configured to recognize, for example by means of the coordinates of the lighting element 51, particular movements of the signal element 5 and to associate them with particular gestures that indicate, for instance, a particular defect.

Once the defect is reported, a laser projector (not illustrated in the figures) projects on the surface of the fabric the outline of the marked defect, in such a way to provide the operator with a feedback.

The processing unit 3, which is in communication with the image detectors 2, is therefore adapted to detect the presence of defects on the basis of the movement of the signal element 5. The processing unit 3 is advantageously configured to associate the defects on the fabric indicated by the signal element 5 with the captured image of the fabric, generating a report indicating the type (i.e. the shape) and the position of the defects on the fabric, as well as the entity of such defects. Still more particularly, it is possible to indicate also the category of the defect, namely if it is a stain, a loose thread and the like. Consequently, the system 1 allows to indicate not only the area and the position, but also the type and the entity of the defects, for example, by means of particular marks/movements by the signal element 5.

Suitably, such report is generated automatically by the processing unit 3.

In other words, on the basis of what has been detected by the operator through the signal element 5, the processing unit 3 is capable of recognizing automatically the type of defect and the entity thereof through the signal element, and to memorize it, and therefore to indicate in the report this information. In particular, advantageously, according to the present invention, the processing unit 3 identifies certain movements of the signal element 5 that correspond to certain types of defects, and reports these defects in the final report.

The coordinates of the pixels of the acquired image refer to a preset bidimensional reference system, on the basis of which the coordinates of the reported defects are calculated.

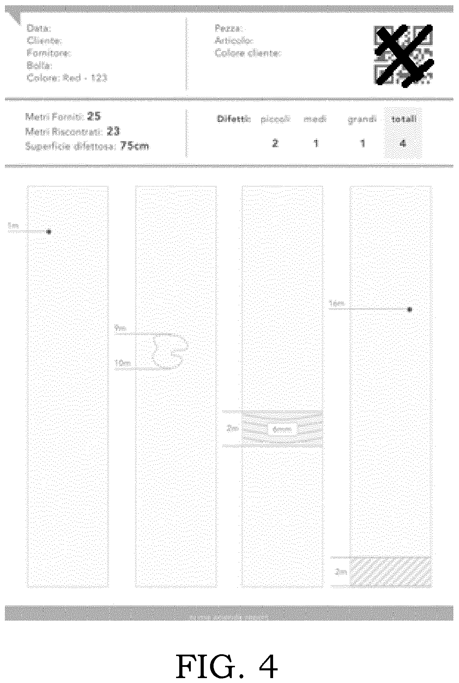

Figure 4 represents an example of report generated automatically by the processing unit 3, in which the type and the position of the defect on the fabric are clearly indicated.

Appropriately, the processing unit 3 is operatively connected with a memory unit, which is configured to store at least the digitalized image of the inspected fabric, as well as the report generated by the processing unit 3. In this manner, it is possible to keep a digital trace of the inspection performed, rendering the generated report available to multiple users in a simple manner.

To that aim, referring again to figure 2, in a preferred embodiment of the present invention, the memory unit is a cloud unit 6 which can be accessed from remote by a user through a specific application and/or a specific address. The acquired images are therefore saved on a server which can be accessed by a user, by means of a device such as a PC or a smartphone, connecting to an Internet address or running a dedicated application installed in its device.

The system 1 of the present invention therefore allows a digital storage of the inspected fabric, making the digitalized image and the report available to multiple users, such as the fabric producer or the company in charge of the specific processing of such a fabric. This system renders much easier possible objections against the producer of the fabric about the quality thereof, since it is possible to access from remote, for example by cloud, the saved image to visualize such a fabric and the report associated therewith. Obviously, it is also possible to save such information locally in a memory unit integrated in the processing unit 3.

A specific identifier is applied to each fabric, such as a label or a bar code, such identifier being associated in a univocal manner with the saved digital image and to the corresponding report. To that aim, in an embodiment of the present invention, the system 1 comprises means for the generation of said bar code and, optionally, means for the automatic application thereof on the fabric.

The digitalization of the fabric and the online storage thereof is particularly advantageous because, besides facilitating objections, it also allows to execute the pricing in a precise manner, as well as to automatize a possible future cutting step (therefore avoiding digitalizing the fabric again), as it will be described below.

To this aim, the processing unit 3 is also configured to define on the digitalized image a plurality of cutting areas, each one corresponding to a certain item to be manufactured by cutting said fabric. The cutting areas are therefore portions of fabric defined by a cutting perimeter that is subsequently followed by a particular cutting instrument inside specifically dedicated numerical control machines. These cutting machines can be arranged downstream the inspection frame to which the system 1 of the present invention is applied, or can be arranged in a remote location, such machines having in their memory the digitalized image and the cutting paths defined thereon. The cutting machines are provided with appropriate readers capable of reading the identifier applied on the fabric during the inspection step, so as to be able to access easily the digitalized image that has been stored in the memory thereof.

Suitably, the number and the position of the cutting areas defined on the fabric is optimized on the basis of the information acquired by the processing unit 3, in particular on the basis of the defects detected. In fact, the processing unit 3 executes the instructions of an algorithm that allows to minimize the waste of fabric, as well as to subdivide the fabric in zones having different characteristics in terms of quality and that are intended to house cutting areas corresponding to different portions of the item to be manufactured and even to different articles.

The system 1, following the digitalization of the image and the generation of the report, is therefore capable, first of all, of performing an estimate of the consumption of fabric based not on theoretical dimensions and other theoretical information anymore, but on real dimensions and other real information (height, length, selvage size, etc.).

Furthermore, as described above, the system 1 is configured to perform automatically the virtual cut of already digitalized rolls, taking into account all the elements (defects, actual position of the logotypes, designs, etc.), such digitalized roll being then subdivided into an optimized number of cutting areas on an offline PC. In this case, we do not speak of "estimate" anymore but of "actual consumption" before the actual cut, significantly saving time and costs, since the optimal cutting mode of the fabric before the placement thereof on a dedicated machine is already known.

Further, through the user interface, it is possible to associate with the reported defects also a particular quality of a zone of the inspected fabric, which is mainly useful for leather cutting. For instance, it is possible to subdivide the object to be inspected into a plurality of zones, to each of which a different quality is assigned. For example, via the user interface it is indicated that a first zone is a high quality one and then the defects in such first zone are reported; afterwards, it is indicated that a second zone is of inferior quality and the defects in this second zone are then identified, and so on. Also this information is stored together with the digitalized images.

Furthermore, for the so-called Jacquard fabrics, striped or squared, i.e. fabrics that have patterns or geometric shapes that are repeated over the fabric (for example, logotypes that are repeated), it is possible to detect automatically the center of each of such repeated elements. Consequently, it is possible to include in the report generated by the processing unit 3 also the error with respect to the material theoretically without errors (for example, a "banana" effect in the weave), as well as to perform also in this case an offline virtual cut of already digitalized rolls, highlighting beforehand (for example, with a video zoom) possible critical issues encountered in the inspection step. For instance, a roll with accentuated "banana" effect can be discarded or preferably used for models of small leather goods in which the problem would be minor. Also in this case, there is the advantage that before the cut all the critical issues of the fabric are already known, which issues can be optimized (for instance, through roto-translations).

Finally, as mentioned before, the system 1 also comprises a user interface 7 such as a touch screen panel, through which the operator can input the necessary information and manage the whole system. In the embodiment illustrated in the figures, the user interface 7 is arranged on the main body 1'.

The present invention also refers to a method for fabric inspection, for example for footwear, leather goods, clothing components and the like, wherein the fabric to be inspected is arranged on a specific inspection frame.

The method of the present invention begins with a preliminary step that provides for the connection of a plurality of image detectors 2 to a pre-existing inspection frame.

Afterwards, the method provides the step of capturing images of the fabric moved by the inspection frame through the image detectors 2, which allow to generate a digital copy of the inspected frame through a processing unit 3 of the acquired images, such processing unit 3 being configured to receive the data acquired by the image detectors 2 and to process such data.

The method of the invention then provides a step of signaling defects on the fabric surface through a signal element such as a digital pen provided with a lighting element that can be detected by the image detectors 2 (or also labels).

Advantageously, the method provides a step of relating, through the processing unit 3, the defects on the fabric indicated by the signal element 5 with the captured image of the fabric.

The method then finishes with the generation of a report indicating the type and the position of the defects on said fabric.

In conclusion, the present invention provides a system capable of being associated with pre-existing inspection frames and capable of capturing a complete digital image of the fabric that is moved in such frames. The system further provides means that allow an operator to indicate the defects present on the fabric without marking the surface thereof. In this way, the system of the present invention is capable of keeping in its memory the captured image of the fabric, together with a detailed report of the defects of such fabric as indicated by the operator, wherein for each defect is automatically reported the type, the entity and the exact position on the fabric according to the indications provided by the operator.

Advantageously according to the present invention, the system proposed allows a complete digitalization of the inspection process of a fabric, with a significant improvement in terms of efficiency and reliability. In particular, the present invention allows to generate a digital map of the defects, which are reported in a simple manner and without errors in the report generated by the processing unit.

The digitalization of the fabric by means of the cameras occurs in a continuous manner in real time, generating a single image file that is stored, together with the report generated, in a cloud memory unit which can be accessed from remote by multiple users, therefore making possible objections much easier.

Consequently, the system of the present invention makes always available the high-resolution digitalized image of the inspected fabric, as well as it makes always available the report that precisely indicates the position and also the shape and the entity of the defects.

All the information acquired in the inspection step can then be used by the system to optimize a future cutting step: basically, it is possible to perform a virtual cut of the digitalized fabric, of which all the necessary information is already known and for which it is therefore possible to indicate, for example, how many items can be manufactured from it. In this case, the processing unit can perform this analysis on the digitalized image before arranging the fabric on a dedicated cutting machine, saving time since the defects of the fabric and therefore the best modality of optimization and cutting are already known. Obviously, also this information can be included in the report generated by the system.

It is therefore evident that the present invention solves the technical problem and allows to obtain the several advantages illustrated above, among which there is a much more efficient and quick inspection, with an error-free report generation.

In particular, the system of the present invention allows to reconfigure in a simple, quick and efficient manner any type of pre-existing inspection frame, without the need to make a new frame.

Obviously, a person skilled in the art, in order to meet particular needs and specifications, can carry out several changes and modifications to the system and method described above, all included in the protection scope of the invention as defined by the following claims.

## Claims

1. System (1) for fabric inspection comprising:
- a main body (1') provided with connection means (1c) for the connection thereof to a pre-existing inspection frame on which fabrics to be inspected are arranged;
- a plurality of image detectors (2) included in said main body (1') and adapted to capture images of a fabric that is moved by the inspection frame;
- a processing unit (3) of the images captured by said image detectors (2), said processing unit (3) being adapted to generate a digital copy of the inspected fabric; and
- a signal element (5) adapted to indicate defects on the fabric surface,
wherein said processing unit (3) is configured to correlate the defects on the fabric indicated by said signal element (5) with the captured image of the fabric, generating a report indicating at least the shape and the position of the defects on said fabric.

2. System (1) according to claim 1, wherein said signal element (5) is provided with a lighting element (51) capable of being detected by the image detectors (2), the coordinates of the movement of said lighting element (51) being calculated by means of said processing unit (3).

3. System (1) according to claim 2, wherein said processing unit (3) is configured to recognize the position, the shape and/or the entity of the defect of the fabric surface on the basis of said coordinates of the movement of the lighting element (5l).

4. System (1) according to claim 2 or 3, wherein said signal element (5) is configured to automatically recognize specific gestures performed by the operator, in such a way to drive said system (1) by means of said specific gestures.

5. System (1) according to any one of the preceding claims, wherein said main body (1') comprises a plurality of lighting elements (4) adapted to illuminate the surface of the fabric to be inspected.

6. System (1) according to any one of the preceding claims, wherein said processing unit (3) is a unit external to said main body (1') and operatively connected therewith.

7. System (1) according to any one of the preceding claims, wherein said processing unit (3) is operatively connected with a memory unit adapted to store at least the digitalized image of the inspected fabric and the generated report indicating the fabric defects.

8. System (1) according to claim 7, wherein said memory unit is a cloud unit which is accessed from remote by a user through a specific application and/or a specific address.

9. System (1) according to any one of the preceding claims, wherein said processing unit (3) is configured to define on said digitalized image a plurality of cutting areas, the type, the number and the position of said cutting areas being defined and optimized according to the information acquired by said processing unit (3) during fabric inspection.

10. System (1) according to any one of the preceding claims, wherein said processing unit (3) is configured to recognize patterns or geometric shapes that are repeated in the fabric, said report further showing the variance in the pattern of the real fabric with respect to that of the theoretical fabric.

11. System (1) according to any one of the preceding claims, wherein said connection means (1c) are configured to connect said main body (1') to the pre-existing frame in a removable manner.

12. System (1) according to any one of the preceding claims, wherein the fabric to be inspected is a fabric of the type for footwear, leather goods, clothing components and the like, wrapped in rolls on the inspection frame, which is configured to unwrap/wrap said fabric during inspection.

13. System (1) according to any one of the preceding claims, wherein said main body (1') comprises a user interface (7).

14. System (1) according to any one of the preceding claims, wherein said main body (1') comprises a laser projector adapted to reproduce on the fabric the outline of the defect reported through said signal element (5), in order to provide the operator with a feedback.

15. System (1) according to claim 1, wherein the signal element (5) is in the form of labels applied at the defect of the fabric to be inspected, said labels being identified by the image detectors (2) and containing information about the type/entity of the defect.

16. Apparatus for fabric inspection comprising means adapted to hold and move a fabric to be inspected, said apparatus including an inspection system (1) according to any one of the preceding claims.

17. Method for fabric inspection, for example for footwear, leather goods, clothing components and the like, comprising the steps of:
- connecting a plurality of image detectors (2) to a pre-existing inspection frame, on which fabrics to be inspected are arranged;
- capturing, by means of said image detectors (2), images of the fabric moved by the inspection frame;
- generating a digital copy of the inspected fabric by means a processing unit (3) of the images captured by said image detectors (2);
- signaling defects on the fabric surface through a signal element (5),
- correlating, through said processing unit (3), the defects on the fabric indicated by said signal element (5) with the captured image of the fabric; and
- generating a report indicating the shape and the position of the defects on said fabric.

18. Method according to claim 17, wherein the signaling of the defects occurs by means of a lighting element or by means of labels.
